# EUROPEAN PATENT APPLICATION

(11) **EP 1 172 122 A1**
(43) Date of publication of application: **16.01.2002**
(21) Application number: 00306025.8
(22) Date of filing: 14.07.2000
(51) Int. Cl.: A61M 15/00

(54) **Dry powder inhaler**

(71) Applicant: THE TECHNOLOGY PARTNERSHIP PUBLIC LIMITED COMPANY, Melbourn, Royston, Hertfordshire SG8 6EE (GB)
(72) Inventor: McDerment, Iain Grierson, Royston, Hertfordshire SG8 6DB (GB); Bowman, Nicholas John, Royston, Hertfordshire SG8 5QW (GB)
(74) Representative: Brunner, Michael John

(57) **Abstract**

A dry powder inhaler device 1 has a flat housing 2A,2B having an air inlet 9,9' and an inhalant outlet 10,10' including a mouthpiece 13. A rotor 4 is provided in the form of a disc having a plurality of recesses 8 for inhalant arranged around its axis of rotation. The rotor is disposed within a chamber 3 in the housing sized to closely fit the disc, and the recesses are indexable, in a single direction of rotation of the disc about its axis relative to the housing, in turn to a dispensing position in which both the air inlet and the outlet are in communication with the recess 8 for the time being located at a dispensing position. Thereby, in use, a pre-metered dose of powdered inhalant within the recess 5 at the dispensing position can be inhaled by a user by being drawn out of the recess entrained in a flow of air through the inlet 9,9', the recess 8 and the outlet 10,10' and mouthpiece 13.

## Description

The present invention relates to inhalers and, more particularly to inhalers which use a dry powder as the inhalant and which provide a predetermined number of pre-metered inhalant doses.

It is known to provide such inhalers in a convenient package size such as to allow users to carry an inhaler in a handbag, pocket or the like. However, conventional inhaler devices, which may be re-usable and, for example, utilise a strip of frangible receptacles each containing a dose of the medicament to be inhaled, the strip being pulled through the inhaler in steps to allow each receptacle to be fractured in use to dispense the medicament, are bulky and, more importantly from a cost and reliability point of view, contain numerous parts which have to be accurately assembled and work consistently over time.

There is therefore a need for an inhaler of this general type which is both simple to assemble and reliable in use, but which is also small.

According to the present invention there is provided a dry powder inhaler device including
a flat housing having an air inlet and an inhalant outlet including a mouthpiece; and
a rotor comprising a disc having a plurality of recesses for inhalant arranged around its axis of rotation, the rotor being disposed within a chamber in the housing sized to closely fit the disc, and the recesses being indexable, in a single direction of rotation of the disc about its axis relative to the housing, in turn to a dispensing position in which both the air inlet and the outlet are in communication with the recess for the time being located at the dispensing position, whereby in use a pre-metered dose of powdered inhalant within the recess at a dispensing position can be inhaled by a user by being drawn out of the recess entrained in a flow of air through the inlet, the recess and the outlet and mouthpiece.

The housing can be approximately credit-card size in area and have a thickness of say 3 to 5mm to allow the inhaler to be easily carried in a pocket, wallet or purse.

A plurality of pre-metered doses of inhalant can therefore be disposed in the recesses for use in a simple and convenient manner by a user.

Preferably, the inhaler has a pair of air inlets and a pair of air outlets, a primary air inlet connecting directly with a primary air outlet and a secondary air inlet connecting with a secondary air outlet via the recess for the time being at the dispensing position, and the secondary air outlet connecting into the primary air outlet.

The use of dry powder requires that ambient moisture be prevented from reaching the pre-metered doses and this may be achieved either by arranging for a sealing fit between the rotor and the walls of the chamber in which the rotor is disposed and by suitable selection of the materials of both the rotor and the housing, or else by providing seals around and/or over the recesses. The recesses may, for example, be provided in one face of the disc and may have a covering foil or similar seal protecting the doses within the recesses and which may either be stripped away by a blade or the like or which may be punctured when the respective recess reaches the dispensing position.

To ensure that the recesses are only indexable in one direction, a ratchet or the like may be provided. This may, for example, be in the form of a spring leg engaging in peripheral recesses in the disc edge or a detent engaging in a face of the disc, the peripheral recesses being shaped such as to allow the leg to ride over the edge of the lands separating the peripheral recesses only in one direction. To allow the user to index the recesses, the edge of the disc may be exposed at one side.

Preferably, the housing has a transparent window at the dispensing position of the recesses allowing the user to confirm the presence of the inhalant dose before using the device and the complete inhalation of the dose after use. This may be provided by manufacturing the housing from a transparent material and masking the surface of the housing (eg by printing) and leaving an unmasked area at the dispensing position of the recesses.

Similarly, it is preferable to provide a dose counter so that the user can see the number of doses taken or remaining. This requirement may be fulfilled by proving indicia on a face of the disc and allowing the indicia to be viewed through the housing at a given location. Again, this may be provided by a transparent housing suitably masked by printing except at the given location.

If desired, the mouthpiece may have a removable or hinged cover and the housing may be formed from a single moulded plastics blank folded around the rotor during assembly.

It will be appreciated that the design of the device according to the invention may include a very small number of parts, significantly reducing material and assembling costs relative to prior inhalers.

A further advantage of this design is the provision of a large flat printing area on the faces of the housing, allowing plenty of room for the printing of instructions, product information and the like.

An example of an inhaler device according to the invention will now be described with reference to the accompanying drawings, in which:

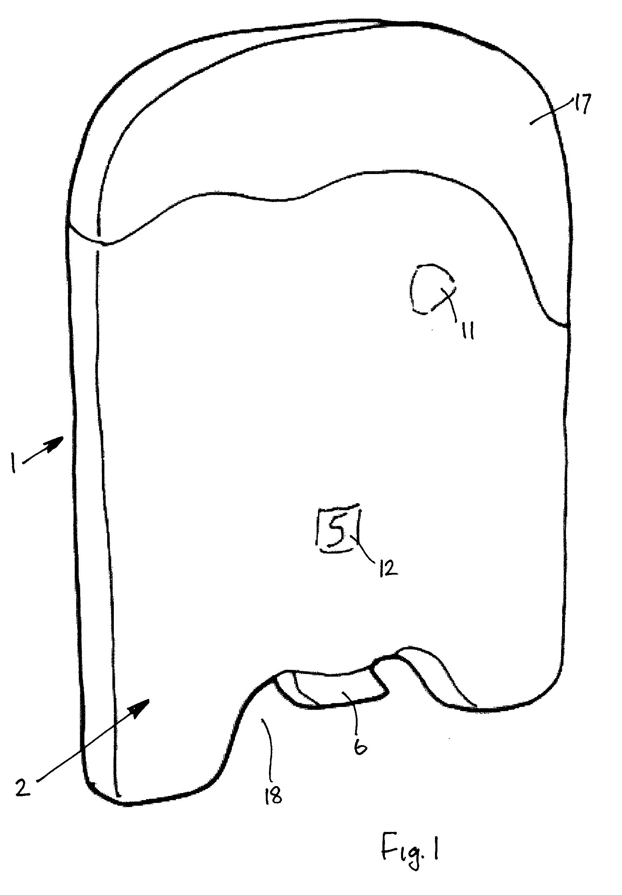

Figure 1 is a perspective view;
Figure 2 is an exploded view;

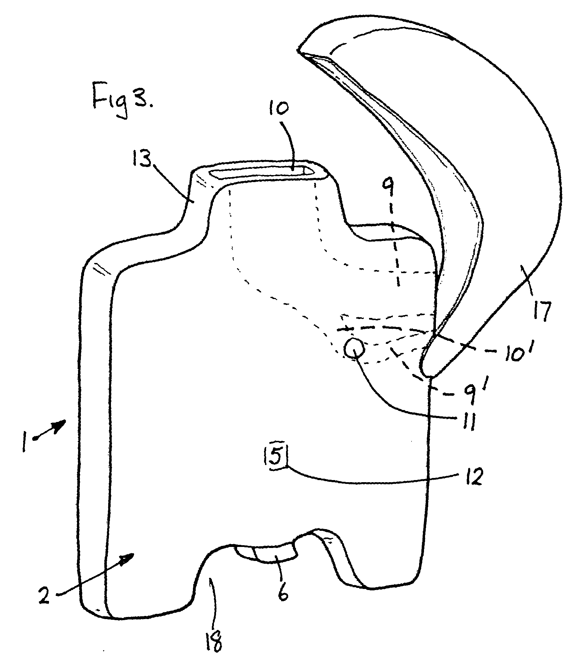

Figure 3 is a perspective view showing some hidden detail; and,

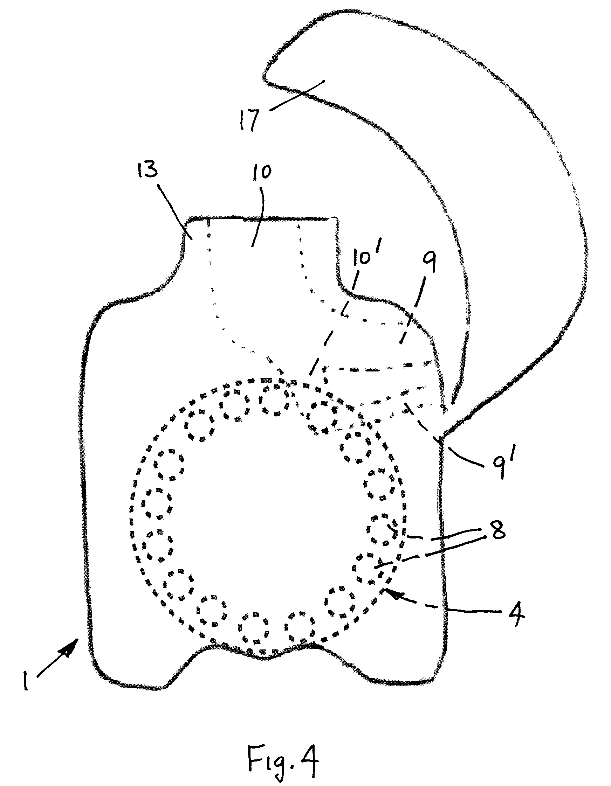

Figure 4 is a front view showing further hidden detail.

In the following description, the same reference numerals will be used for the same or similar parts in both devices.

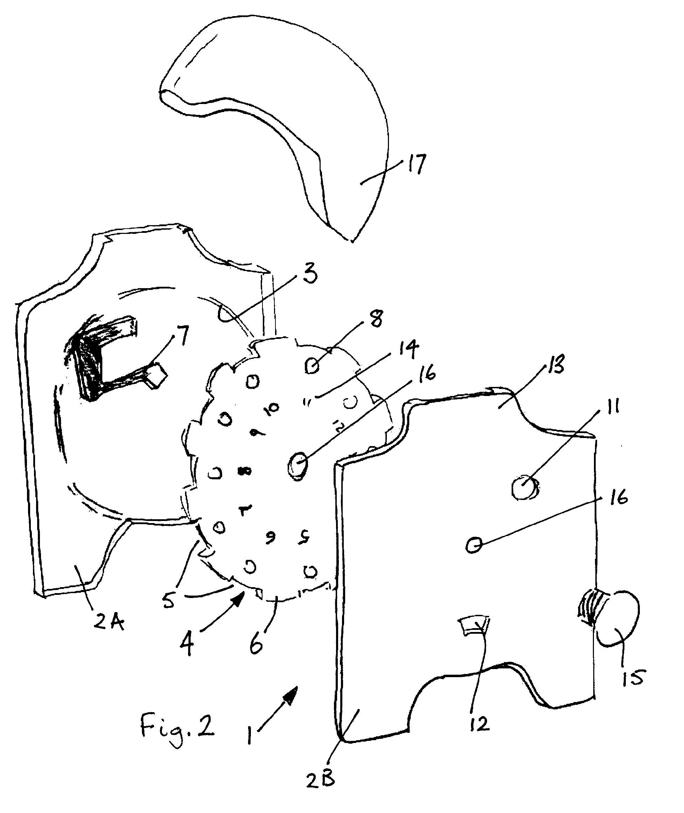

The inhaler 1 shown in the figures has a transparent polypropylene or other plastics housing 2 which, for the sake of convenience is shown, in figure 2, in two parts 2A,2B, but which may be formed from a single, hinged blank. The housing is approximately credit-card size for convenience and has, in one half 2A, a generally circular chamber 3 which contains a disc like polypropylene (or similar) rotor 4 with a castellated edge providing plural edge recesses 5 separated by lands 6. A spring leg 7 disposed in the housing 2, or formed integrally with it, provides a ratchet action by engagement of one end in the edge recesses in turn as the disc like rotor is rotated about its axis. To facilitate this the edge recesses 5 are chamfered on one side to allow the spring leg 7 to ride out of the recesses over the adjacent land when the rotor 4 is turned in one direction.

The disc like rotor has inhalant dose recesses 8 disposed peripherally around the rotor disc 4 in one face 4A of the rotor, the number of inhalant dose recesses equalling the number of edge recesses 5 and being positioned so that each recess in turn is registrable with air inlet and outlet channels 9',10' formed in the housing half 2B. In an alternative form of the device, the air channels may be formed between the housing halves 2A,2B.

Primary and secondary air inlet channels 9,9' and outlet channels 10,10' are preferably provided so that a primary inlet airflow passes through a primary inlet channel 9 and into the main or primary outlet channel 10, with a secondary airflow through the inlet channel 9' and secondary outlet channel 10', the flow through the secondary inlet channel 9' and a secondary air outlet channel 10' being arranged to pass through the recess at the dispensing position, caused by the flow through the primary air inlet channel 9 and primary outlet channel 10 and the resulting pressure drop at the junction with the secondary air outlet channel 10'. The advantage of this is that powder inhalant is fed gradually into the outlet airstream. Depending on the specific use of the inhaler, the target users, the specific inhalant etc., the relative sizes of the channels may be altered accordingly. The main outlet channel 10 extends into a mouthpiece 13.

The exterior face of the housing half 2B is printed so as to reveal a windows 11 which is positioned over the junction of the inlet 9' and subsidiary outlet channel 10' and at a position overlying the dispensing position of each of the recesses in turn. This allows the pre-metered dose of medicament arranged in each dose recess 8 to be checked before use and enables the user to check that all the medicament has been inhaled from the recess 8 at the dispensing position.

A further window 12 is provided located at a position overlying a series of numeric indicia 14, acting as does usage counters, printed on the disc rotor 4. These can be viewed through the window 12 to allow a user to check the number of doses either remaining or used.

The rotor may be held in position by a circular rivet or plug 15 which locates through opposed holes 16 in the housing halves 2A,2B and the rotor 4. The rotor disc 4 may be biassed into engagement with the housing half 2B by a coil spring or the like (not shown) in order to seal the recesses 8 against ambient moisture. Alternatively, not shown, a foil seal may be provided over each recess 8, for example, and being stripped away by a stripper as the recess approaches the dispensing position. A hinged cover 17 covers the mouthpiece 13 until the inhaler is used, at which time it can be pivoted away from the mouthpiece.

To allow a user to rotate the rotor disc 4, the housing 2 is cut away on the side opposite the mouthpiece 13, providing a hollow 18 through which the lands 6 of the rotor disc 4 pass and can be engaged by a user's finger or thumb.

## Claims

1. A dry powder inhaler device including
a flat housing having an air inlet and an inhalant outlet including a mouthpiece; and
a rotor comprising a disc having a plurality of recesses for inhalant arranged around its axis of rotation, the rotor being disposed within a chamber in the housing sized to closely fit the disc, and the recesses being indexable, in a single direction of rotation of the disc about its axis relative to the housing, in turn to a dispensing position in which both the air inlet and the outlet are in communication with the recess for the time being located at a dispensing position, whereby in use a pre-metered dose of powdered inhalant within the recess at the dispensing position can be inhaled by a user by being drawn out of the recess entrained in a flow of air through the inlet, the recess and the outlet and mouthpiece.

2. A dry powder inhaler device according to claim 1, wherein the housing is substantially credit-card size in area.

3. A dry powder inhaler device according to claim 1 or claim 2, wherein the housing has a thickness of between 2 and 5 mm.

4. A dry powder inhaler device according to any of claims 1 to 3, wherein the rotor and walls of the chamber are a sealing fit.

5. A dry powder inhaler device according to any of claims 1 to 3, including seals around and/or over the recesses.

6. A dry powder inhaler device according to any of claims 1 to 5, including a ratchet or detent to allow the rotor to rotate in one direction but prevent its rotation in the other direction.

7. A dry powder inhaler device according to any of claims 1 to 6, wherein, to allow the user to index the recesses, the edge of the disc is exposed through the housing.

8. A dry powder inhaler device according to any of claims 1 to 7, wherein the housing has a transparent window at the dispensing position of the recesses allowing the user to confirm the presence of the inhalant dose before using the device and the complete inhalation of the dose after use.

9. A dry powder inhaler device according to any of claims 1 to 8, including a dose counter so that the user can see the number of doses taken or remaining.

10. A dry powder inhaler device according to claim 9, wherein the dose counter comprises indicia on a face of the disc and a window in the housing allowing the indicia to be viewed through the housing at a given location.

11. A dry powder inhaler device according to claim 8 or claim 9, wherein the window is provided by forming the housing of a transparent material, masking the surface of the housing and leaving a portion of the housing unmasked at the desired window location.

12. A dry powder inhaler device according to any of claims 1 to 11, wherein the mouthpiece has a removable or hinged cover.

13. A dry powder inhaler device according to any of claims 1 to 12, wherein the housing is formed from a single moulded plastics blank folded around the rotor during assembly.

14. A dry powder inhaler device according to any of claims 1 to 13, including a pair of air inlets and a pair of air outlets, a primary air inlet connecting directly with a primary air outlet and a secondary air inlet connecting with a secondary air outlet via the recess for the time being at the dispensing position, and the secondary air outlet connecting into the primary air outlet.
